# EUROPEAN PATENT SPECIFICATION

(11) **EP 1 751 661 B1**
(45) Date of publication and mention of the grant of the patent: **15.12.2010**
(21) Application number: 05738858.9
(22) Date of filing: 05.05.2005
(51) Int. Cl.: C12N 5/074

(54) **METHOD FOR SELECTIVELY EXPANDING, SELECTING AND ENRICHING STEM/PROGENITOR CELL POPULATIONS**
VERFAHREN ZUM SELEKTIVEN EXPANDIEREN, AUSWÄHLEN UND ANREICHERN VON STAMM- BZW. VORLÄUFERZELLEN-POPULATIONEN
PROCEDE DE CROISSANCE, DE SELECTION ET D'ENRICHISSEMENT SELECTIFS DE POPULATIONS DE CELLULES SOUCHES/CELLULES PROGENITRICES

(30) Priority: 06.05.2004 US 568248 P
(43) Date of publication of application: 14.02.2007
(73) Proprietor: Tel HaShomer Medical Research Infrastructure and Services Ltd., 52 621 Ramat Gan (IL)
(72) Inventor: TREVES, Avraham, J., 90805 Mevaseret Zion (IL); GALSKI, Hanan, 97850 Jerusalem (IL); BAR, Iris, 71700 Modi'in (IL); NAGLER, Arnon, 96411 Jerusalem (IL)
(74) Representative: Modiano, Micaela Nadia
(86) International application number: PCT/IL2005/000475
(87) International publication number: WO 2005/109199

(56) References cited:
- WO-A-99/61589
- BUNTING K D ET AL: "Transduction of murine bone marrow cells with an MDR1 vector enables ex vivo stem cell expansion, but these expanded grafts cause a myeloproliferative syndrome in transplanted mice" BLOOD, W.B. SAUNDERS, PHILADELPHIA, VA, US, vol. 92, no. 7, 1 October 1998 (1998-10-01), pages 2269-2279, XP002123732 ISSN: 0006-4971
- GALSKI H ET AL: "A novel MDR-dependent pharmacological approach for ex-vivo expansion of haematopoietic stem cells from human umbilical cord blood" BONE MARROW TRANSPLANTATION, vol. 39, no. Suppl. 1, April 2007 (2007-04), pages S126-S127, XP009096178 & 33RD ANNUAL MEETING OF THE EUROPEAN-GROUP-FOR-BLOOD-AND-MARROW-TRANSP LANTATION; LYON, FRANCE; MARCH 25 -28, 2007 ISSN: 0268-3369
- WAGNER WOLFGANG ET AL: "Molecular evidence for stem cell function of the slow-dividing fraction among human hematopoietic progenitor cells by genome-wide analysis" BLOOD, vol. 104, no. 3, 1 August 2004 (2004-08-01), pages 675-686, XP002469422 ISSN: 0006-4971
- DATABASE MEDLINE [Online] TSUNG ET AL: 'Expression of human multidrug resistance gene (mdr1) cDNA in murine ES cells and in chimeric mice', XP008093339 Retrieved from STN Database accession no. 7912874 & SHI YAN SHENG WU XUE BAO vol. 26, no. 4, December 1993, pages 429 - 439, XP003001773
- CENTOLA ET AL: 'The gene for familial Mediterranean fever, MEFV, is expressed in early leukocyte development and is regulated in response to inflammatory mediators' BLOOD vol. 95, no. 10, 15 May 2000, pages 3223 - 3231, XP003001771
- DATABASE MEDLINE [Online] BERTOLINI ET AL: 'Retrovirus-mediated transfer of the multidrug resistance gene into human haemopoietic progenitor cells', XP008093340 Retrieved from STN Database accession no. 7803276 & BRITISH JOURNAL OF HAEMATOLOGY vol. 88, no. 2, October 1994, pages 318 - 324, XP003001774

## Description

### FIELD AND BACKGROUND OF THE INVENTION

The present invention relates to production of stem cells and, more particularly, to a method of in vitro enrichment of stem cells in a population of cells

### 1. The need

Successful ex vivo expansion of stem and progenitor cells could be exploited for a variety of clinical applications. For exaple, such applications include increasing the number of stem cells available for genetic modification and bone marrow transplantation as well as the generation of large quantities of immunologically reactive cells (T cells, natural killer cells, dendritic cells, and others) for adoptive immunotherapeutic purposes (**Devine 2003**). Studies to date have not addressed whether true hematopoietic stem cells with long-therm repopulating potential can be expanded in culture, and transfused to shorten the period of pancytopenia and improve the overall rate of engraftment. In the light of this, there is a critical need in the invented method for ex vivo expansion of enriched stem cell populations.

The invented process will be suitable for both autologous and allogeneic transplantations as it will result in a reduction of the volumes of transplants to be cryopreserved. In addition, a smaller scale immnunoselection technique, requiring less monoclonal antibody can be used for final purification after stem cells have been enriched by the invented procedure.

The invented method may be also useful in the ex vivo expansion of the following stem cells: bone marrow, peripheral blood, umbilical cord blood, myoblasts, cardiomyoblasts, hepatic stem cells, neural-stem cells, mesenohymal stem cells, endothelial stem cells, non-human embryonic stem cells, fetal stem cells or any other type of pluripotent stem cells that express this property (i.e., relatively higher resistance to cytostatic/cytotoxic agents).

For example, in treatment of cardiac insufficiency ex. vivo expanded stem cells could be accessible as single cell types with ideal properties as donor cells (**Perry 2003**).

In addition, bone marrow and umbilical cord blood derived stem cells have been used in pre-clinical models of brain injury, directed to differentiate into neural phenotypes, and have been related to functional recovery after engraftment in central nervous system (CNS) injury models (**Newman 2003**). Therefore, the suggested technique for stem cell enrichment may be combined also in cell-based transplantation therapies for neurodegenerative disorders.

Its use will also be advantageous in stem cells- and cancer- research and clinical applications due to its lower cost as compared to stem cells enrichment techniques based on antibodies.

### 2. Background

### 2.1 Stem cells transplantation

Hematopoietic progenitor cells can be mobilized from the bone marrow (BM) to the blood by a wide variety of stimuli, including hematopoietic growth factors, chemotherapy, and chemokines. Moreover, it was observed that the mobilizing effect of chemotherapy can be enhanced by in vivo administration of hematopoietic growth factors, such as granulocyte colony-stimulating factor (G-CSF). Increasingly, mobilized peripheral blood (PB) hematopoietic progenitor cells instead of BM hematopoietic progenitor cells have been used to reconstitute hematopoiesis after myeloablative therapy because of their reduced engraftment times and relative ease of collection **(Thomas 2002).** In allogeneic PB stem cells transplantation compared with allogeneic BM transplantation, the incidence and frequency of graft-versus-host (GVHD) is of concern because high number of T lymphocytes are infused in allogeneic PB stem cells transplantation. The incidence arid severity of acute GVHD are not increased but chronic GVHD is higher in allogeneic PB stem cells transplantation compared with allogeneic BM transplantation **(Kasai 2002).**

Umbilical cord blood (UCB) has been rapidly established as an alternative source of stem cells to BM for allogeneic-related and unrelated hematopoietic transplantation. The main advantage of UCB transplantation is the relative ease of procurement and the lower-than-anticipated risk of severe acute GVHD **(Koh 2004).** The use of reduced-intensity or nonmyeloablative preparative regimens to allow engraftment of UCB broadens the scope of patients who may benefit from allogeneic immunotherapy, including elderly and medically infirm patients with no matched sibling donor. To date, about 200,000 UCB units are available for transplantation and more than 3500 UCB transplants have been performed, mostly in children, for the treatment of a variety of malignant and nonmalignant conditions **(Benito 2004).** The major disadvantage of UCB is the low yield of stem cells, resulting in higher graft failure rates and slower time to engraftment compared to BM transplantation. A rational approach thus involves ex vivo expansion of UCB-derived hematopoietic precursors **(Cohen 2004).**

Therefore, our novel method of stem cell expansion will be demonstrated on UCB-derived hematopoietic stem cells, although it may be also suitable for non-hematopoietic stem cells, e.g., mesenchymal stem cells that are an attractive therapeutic tool for cell transplantation and tissue engineering as well **(Iris 2003).**

### 2.2 Multidrug resistant -1 (MDR1) and ABCG2 gene expression and resistance to cytostatic and/or cytotoxic agents.

Resistance to various chemically different natural product, anti-cancer drugs (multidrug resistance, or MDR) results from over-expression of one or more ATP-dependent efflux transporters and subsequent decreased drug accumulation. The first of these to be identified was P-glycoprotein (Pgp), the product of the human MDR1 ***(ABCB1)*** gene, localized to chromosome 7q21. Pgp is a member of the large ATP-binding cassette (ABC) family of proteins **(Ambudkar 2003, Findling-Kagan 2005, Galski 2003, Sauna 2001).** ABCG2 (BCRP1) is another member of the ABC family of cell surface transport proteins, located on chromosomal locus 4q22 **(Abbott 2003).** The substrate profiles of Pgp (MDR1) and ABCG2 transporters include various cytostatic agents and antineoplastic drugs

### 2.3 Stem cells have relative high expression level of MDR1 and ABCG2.

There are several indications that stem cells express MDR1 in relatively high levels. For example **Bertolini 1997** showed that after drug exposure most of the peripheral blood progenitor cells displayed a CD34⁺, CD38⁺, MDR1⁺, Rhodamine 123 (Rh123) low and Hoechst 33342 (Ho) low phenotype, and as few as 180 of these drug-resistant cells were able to generate a stable multilineage human hematopoiesis in sublethally irradiated immunodeficient mice. Recently **Uchida 2004** demonstrated that activation of adult hematopoietic stem cells (HSC) in vivo following 5-fluorouracil treatment, or in vitro with cytokines, induces variable losses in Rh123 and Ho efflux activities. Moreover, HSC from MDR1a/1b(-/-) mice show a dramatic decrease in Rh123 efflux ability. The supravital dyes Rh123 and Ho are powerful probes for the characterization, resolution, isolation, and purification of primitive HSC. The fidelity of Rh123 and Ho as stem cell probes resides in their individual and combined ability to hierarchically order the HSC on the basis of their probability of cycling by probing individual traits that define the quiescent state, and by exploiting the overlapping activity of transmembrane efflux pumps belonging to the ABC transporter superfamily, including MDR1,MRP1, and BCRP1/ABCG2, for which they are preferential substrates **(Bertoncello 2004).** ABCG2 expression was shown within a more primitive subpopulation of cells than MDR1 **(Zhou 2001).**

Recent studied also indicate that non-hematopoietic stem and/or progenitor cells express MDR1 in relatively high levels. Study of **Fiaccavento 2005** demonstrated the presence of an increased number of c-kit positive, NDR-positive, and Sea-1-positive stem cells within the myocardium of hereditary delta-SG null hamsters, a spontaneously occurring model of hypertrophic cardiomyopathy. Moreover, hepatic progenitor cells of rats treated with 2- acetylaminofluorene followed by partial hepatectomy express high levels of active multidrug resistance protein 1 (MRP1) and MRP3, as determined by real time detection RT-PCR **(Ros 2003).** MRP1,MRP2 and MRP3 also belong to the ABC transporter family.

### 2.4 Surface antigens expression of HSC.

Prominin, also termed CD133 (AC133), is a highly conserved antigen expressed on HSC associated with mobility and primitive function. **Koehl 2002** reported for the first time a successful transplantation with a CD133 positive, selected graft. The fact that CD133⁺ hematopoietic progenitors can give rise to an adherent population which is CD133⁻ and CD34⁻ and that these cells can again give rise to a CD133⁺CD34⁻stem cell population with high NOD/SCID engraftment potential **(Handgretinger 2003),** indicates that CD133 might be an earlier marker of hematopoietic precursors than CD34. **Dimitriou 2003** observed a more effective proliferation of the CD34⁺ population than the CDT33⁺ population, while the CD133⁺ cell fraction retained and expanded more immature elements. Therefore, they concluded that CD133⁺ and CD34⁺ expanded UBC cells could potentially be used in combination to overcome the shortcomings of cord blood transplantation in older children and adults **(Dimitriou 2003).** Recently, **Forraz 2004** isolated a discrete lineage-negative (Lin⁻) cell population that maintained/expanded more primitive hematopoietic stem and progenitor cell than CD133⁺ cells but underwent slow proliferation. Lately **Wagner 2004** showed that the slow dividing fraction of hematopoitic progenitor cells associated with primitive function and self-renewal is characterized by a highest expression level of CD133 and MDR1 genes as detected by microarray analysis. This study strengthens our rational that treatment with cytostatic and/or cytotoxic agents could selectively affect mature and differentiated cells (MDR1/ABCG2 negative) while supporting the growth of stem cells (MDR1/ABCG2 positive).

### 2.5 Current techniques for stem cell enrichment:

Several immunological techniques have been developed for the isolation of stem cells. In most of the available methods, CD34 or CD133 monoclonal antibodies (mAbs), which react specifically with hematopoietic stem cells, are being used for the isolation of progenitor cells.

One major disadvantage of the available techniques is the large amount of mAbs needed, which makes these techniques very expensive. A second disadvantage is the need of some technique to release the cells, which may result in damage to the progenitor cells. The use of affinity columns to separate stem cells is difficult when frozen cells are used (e.g., frozen cord blood units), mainly due to aggregation of the thawed cells. Therefore, a process leading to substantial enrichment of stem cells by depletion of non-stem cells, without the constant use of mAbs or affinity columns would be a considerable advantage.

Several methods have also been reported for ex vivo expansion of stem cells, but these methods always require a step of immuno-selective enrichment of the stem cells before and/or during expansion **(Gammaitoni 2003, Petzer 1997, Peled 2002).**

### SUMMARY OF THE INVENTION

The present invention is a procedure for enrichment and selective expansion of stem cells (excluding human embryonic stem cells) and/or progenitor cells based on selective killing (or growth arrest) of MDR1 negative non-progenitor cells by treatment with a combination of cytostatic and/or cytotoxic reagent together with a cocktail of growth factors. These conditions will selectively kill mature and differentiated cells (MDR1/ABCG2/MRP negative) while supporting the growth of stem cells (MDR1/ABCG2/MRP positive).

According to the present invention there is provided an in vitro method for producing a population enriched for stem/progenitor cells, the population including stem/progenitor cells that inherently overexpress at least one transporter gene selected from the group consisting of an ABCB1 (MDR1) gene and/or an ABCG2 gene and/or MRP1 gene and/or MRP2 gene and/or MRP3 gene relative to the more differentiated progenitor and mature cells of the population, said method comprising: treating said population with at least one first agent to which stem/progenitor cells are less sensitive than are other cells in said population, said first agent selected from the group consisting of cytostatic agents and cytotoxic agents, said first agent selectively depleting from the population said more differentiated progenitor and mature cells, thereby producing a population enriched for stem/progenitor cells, wherein said population is not obtained from a human embryonic source.

According to the present invention there is provided a method of producing stem cells, including the steps of: (a) providing a population that includes the stem cells; and (b) treating the population with at least one first agent, selected from the group consisting of cytostatic agents and cytotoxic agents, to which the stem cells are less sensitive than other cells in the population.

The population may be obtained from an embryonic source, from a fetal source or from an adult source.

Preferably, the population, is obtained from bone marrow, blood (e.g., peripheral blood or umbilical cord blood), fat, muscle, skin or any other tissue as neural tissue or tissue from internal organs such as the heart, the intestine, a kidney, the liver, a lung or the pancreas.

Preferably, the first agent(s) selectively deplete(s) cells that are negative with respect to expressing a transporter gene of the first agent(s) while tending to spare cells that are positive with respect to expressing that gene. Examples of such transporter genes include members of the ABC transporter gene family such as MDR1,ABCG2, or members of the MRP transporter gene family such as MRP1, MRP2 and MRP3.

Preferably, the method also includes the step of treating the population with one or more cytokines or growth factors.

The scope of the term "stem cells" as used in the appended claims includes both stem cells and progenitor cells.

### BRIEF DESCRIPTION OF THE DRAWINGS

The invention is herein described, by way of example only, with reference to the accompanying drawings, wherein:

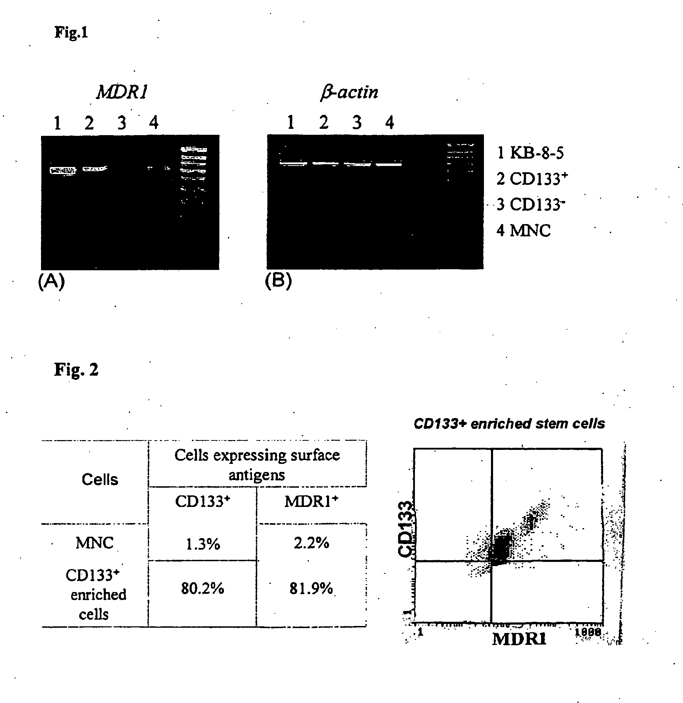

FIG. 1 shows expression (RT-PCR) of (A) MDR1- and (B) β-actin- RNA in UCB-derived CD133+ cells relative to CD133- and mononuclear cells (MNC). RNA was extracted from: 1, KB-8-5 cell line (MDR1 positive control); 2, fresh enriched CD133-positive cell fraction; 3, CD133 negative-cell fraction; 4, MNC isolated by density gradient centrifugation.
FIG. 2 shows flow cytometry (FACS) analyses for Pgp (MDR1) expression of MNC and CD133+ enriched cells (CD133 positive fraction) isolated form fresh UCB.. A representative analysis is shown.

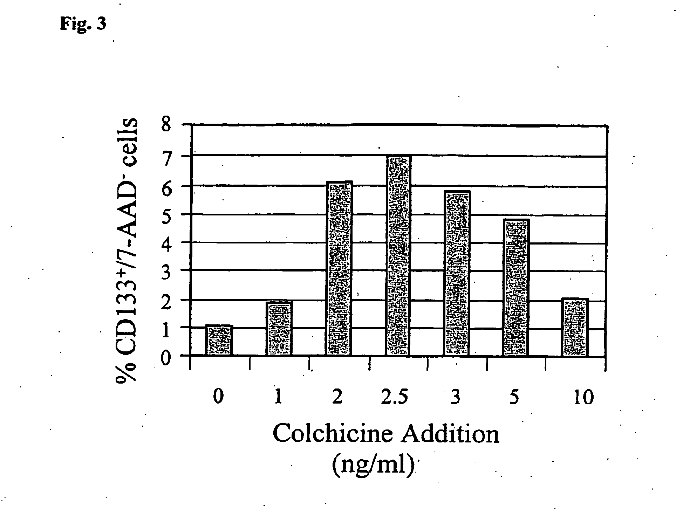

FIG. 3 is a bar graph of dose-dependent enrichment of UCB-derived CD133+ cell-fraction by cytostatic agent addition to expansion medium. Representative results from flow cytometry analyses after 2 weeks expansion in culture are shown. Results are indicated as % of CD 133 positive cells from total viable cells (7-AAD unstained cells).

### DESCRIPTION OF THE PREFERRED EMBODIMENTS

The present invention is of a process for the preparation of enriched populations of stem/progenitor cells (wherein said population is not obtained from human embryonic source) for various cell therapy applications, without the continuous use of xenogeneic antibodies directed against cell surface molecules, affinity columns or density gradients. The invention includes a method for enrichment and expansion of stem/progenitor cells based on their relatively high expression levels of multidrug resistant -1 (MDR1, also termed ABCB1) gene and/or ABCG2 gene and/or MRP gene, as compared to more differentiated progenitor and mature cells. The invention relates to expansion of stem/progenitor cells in vitro (in the presence of cytokines and growth factors) using selective killing of MDR1 and/or ABCG2 and/or MRP low/negative cells by cytostatic and/or cytotoxic agents while avoiding the killing of MDR1 and/or ABCG2 and/or MRP positive stem/progenitor cells. The principles and operation of stem/progenitor cell enrichment according to the present invention may be better understood with reference to the drawings and the accompanying description.

### 3. Example:

### 3.1 Methods:

### 3.1.1 Cord blood samples

Cells were obtained from human umbilical cord blood (UCB) after normal full-term delivery. The cells were layered on a Ficoll-Hypaque gradient. (1.077 g/ml Sigma), and centrifuged at 500g for 30 minutes. The mononuclear cells in the interface layer were collected and washed three times in phosphate-buffered saline (PBS; Biological Industries) containing 0.5% Bovine serum albumin (BSA). To purify the CD133⁺ cells, the mononuclear cell fraction was subjected to two cycles of immunomagnetic bead separation using a MiniMACS CD133 progenitor cell isolation kit (Miltenyi Biotec,), according to the manufacturer's recommendations.

The purity of the CD133⁺ population thus obtained was evaluated by flow cytometry (see section 3.1.3).

### 3.1.2 Ex vivo expansion

Purified CD133⁺cells were cultured in 24-well culture plate at densities of 2-4 X10⁴ cells in alpha-MEM medium (Biological Industries) supplemented with 10% fetal calf serum (Biological Industries) and the following human recombinant cytokines (Pepro Tech, Inc., Rocky Hill, NJ, USA): Trombopoietin, interleukin-6, FLT-3 ligand and Stem Cell Factor, at final concentration of 50 ng/ml each as well as interleukin-3 and IL2 at 20 ng/ml, in the absence or presence of colchicine in a concentration scale as indicated. Cells were expanded at 37°C in a humidified atmosphere of 5% CO₂ in air.

Cultures were depopulated twice a week and a fresh medium in which growth factors and colchicine were added. At various time points, cells were counted after staining with trypan blue and harvested cells were used for enumeration of CD133⁺ cells following immunophenotype analysis.

### 3.1.3 Flow cytometry for analyses of surface antigens

The cells were washed with a PBS solution containing 1% BSA and stained (at 4°C for 30 min) with fluorescein isothiocyanate (FITC)- or phycoerythrin (PE)-conjugated antibodies that are specifically described in the next sections. Cells were washed in the abovementioned buffer and analyzed by flow cytometer (Becman coulter). Cells were passed at a rate of up to 1000 cells/second, using a 488-nm argon laser beam as the light source for excitation.

Cells stained with FITC- and PE-conjugated isotype control antibodies were used to determine background fluorescence.

### 3.1.4 Determination of CD133⁺cell subset after expansion

The percentage of CD133⁺ cell fraction from total cells was determined by flow cytometry using. PE-anti- CD133⁺ antibody (Miltenyi Biotec). Fold enrichment was calculated by dividing the CD133⁺ cell percentage of culture growing with cytostatic agent by the CD133⁺ cell percentage of culture growing without cytostatic agent. Since cultures were depopulated twice a week, the culture volume was calculated by multiplying the actual volume with the number of passages. Fold expansion was calculated by dividing the CD133⁺ cell content of the culture by the initial number of cultured CD133⁺ cells.

### 3.1.5 Determination of early stem cell subsets

The percentages of early stem cell subsets were determined from the purified: fresh and/or cultured CD133⁺cell fraction. Cells were dually stained with PE anti-CD34 and FITC anti-CD38 (DAKO) for determination of CD34⁺CD38˙ cells by flow cytometry analyses with PE anti- CD133⁺ antibody (Miltenyi Biotec).

### 3.1.6 Determination of Pgp (MDR1) surface expression in stem cell subsets

Cells were dually stained with PE anti- CD133⁺ antibody (Miltenyi Biotec) and with MRK16 antibody against Pgp and FITC secondary goat anti mouse antibody (Jackson). Stained cells were subjected to FACS analyses.

### 3.1.7 Preparation and analysis of RNA

Total RNA was isolated using RNeasy Mini kit (Quiagen Sciences, Germantown, MD, USA), according to the manufacturer instructions. RNA expression was measured by RT-PCR. cDNA was synthesized by reverse transcription system (Promega, Madison, WI, USA), according to manufacturer instructions.The cDNA was used as a template for subsequent amplification of the human MDR1 gene transcript. Amplification of the *β-actin* gene was used as internal control.
RT-PCR was performed using the primers,
F: 5'-TTTACTGATAAAGAACTCTTA-3'
R: 5'-AACTGAAGTGAACATTTCTG-3' for human *MDR1,* (product size 487 bp) and the primers
F: 5'- CCAAGGCCAACCGCGAGAAGATGAC-3'
R: 5'-AGGGTACATGGTGGTGCCGCCAGAC-3'.for β*-actin* (product size 589 bp).

Reaction mixtures contained the following: 0.5 µg cDNA, 25pM of each primers and 2X ReddyMix PCR Master Mix (ABgene, Surrey, UK), which contains 1.25U Thermoprime Plus DNA Polymerase, 75mM Tris-HCl (pH 8.8), 20mM (NH₄)₂SO₂, 1.5mM MgCl₂, 0.01% Tween 20, 0.2mM of each dNTP, precipitant and red dye for electrophoresis. Cycling parameters were: denaturation in 94°C for 1 min, annealing in 61_{°}C for 1 min, and extension in 72°C for 1 min. Samples were cycled using a Paltier Thermal Cycler, PTC-200, (MJ Research, Watertown, MA, USA).

The RT-PCR products were separated by 2% agarose gel electrophoresis and visualized under UV light using ethidium bromide staining. Gels were scanned and analyzed by EDAS 290 Electrophoresis Documentation and Analysis System (Kodak).

### 3.2 Results

### 3.2.1 Selection and characterization of enriched stem cell population

Enriched CD133⁺ stem cell population was prepared from human, UCB-derived MNC by CD133 immunomagnetic beads isolation kit. The *MDR1* expression level of UCB-derived CD133⁺ cells in comparison to UCB-derived MNC and CD133⁻ cells was studied using RT-PCR and flow cytometry (Figure 1 and 2, respectively). As shown in Figure 1A, *MDR1* RNA expression level in UCB-derived CD133⁺ cells is significantly higher than its .. expression in both UCB-derived MNC and CD133⁻ cells. The expression level of the housekeeping gene, β-action, was similar in all the tested sub-populations (Figure 1B). The purity of CD133⁺ cells was measured by flow cytometry (Figure 2). Results indicated that approximately 80% of the CD133 enriched cells were positive for CD133 and for Pgp (MDR1) antigens. Pgp expression in UCB derived CD133⁺ cells is significantly higher than its expression in MNC (81.9% v.s. 2.2%, respectively). Moreover, most of the UCB-derived CD133⁺ cells also express Pgp. These results indicate that CD133⁺ stem cells express higher level of MDR1 relatively to the other cells and, therefore, may be further selected by cytostatic agents to enrich their fraction.

### 3.2.2 Effect of cytostatic agent treatment on enrichment and expansion of CD133⁺, stem cell population

purified UCB-derived CD133⁺ cells were cultured in cytokine-supplemented liquid medium in the continuous presence or absence of cytostatic agent (colchicine) in a concentration scale (Figure 3). Flow cytometry analysis after 2 weeks expansion (of three independent experiments) demonstrated a dose-dependent enrichment of UCB-derived CD133⁺ cell-fraction by the cytostatic agent colchicine and revealed the optimal concentration of 2.5 ng/ml in which the highest CD133⁺ cells enrichment was achieved (Figure 3).

Although intrinsic variability was demonstrated in the ex vivo expansion potential of CD133⁺ cells derived from different cord blood donors, the use of cytostatic agent substantially increased the renewal potential, resulting in prolongation of CD133⁺ cell enrichment and expansion (Table 1 and Table 3).

### 3.2.3 Effect of cytostatic agent treatment on early progenitors

CD34⁺ hematopoietic progenitors comprise a heterogeneous population. The minority, CD34⁺CD38⁻ are lineage uncommitted progenitors, whereas the majority, CD34⁺CD38⁺ cells, are lineage committed cells.

To determine the effect of cytostatic agent on CD34⁺CD38⁻ early stem cell sub-population, purified UCB derived CD133⁺ cells after 1 week in culture in the absence or presence of colchicine were analyzed by flow cytometry for CD34 and CD38 surface antigen expression. The results (summarized in table 2) indicated an increase in the percentage of CD34⁺CD38⁻ sub-population in cultures treated with cytostatic agent comparing to un-treated cultures. These results demonstrated that colchicine enabled preferential proliferation of early progenitor cells with CD133⁺ and CD34⁺38⁻ phenotype, resulting in the observed increased ex vivo expansion.

**Table 1: Enrichment of CD133⁺ stem cell population in culture in absence and presence of cytostatic agent. Results obtained after expansion of CD133 positive cells for two weeks in the absence or presnece of cytostatic agent (colchicine) at 2.5 ng/ml. Results from three independent experiments of different UCB donors are shown.**

| Experiment | CD133+ cell population *without* cytostatic agent | CD133+ cell population *with* cytostatic agent | Fold enrichment |
|---|---|---|---|
| Donor # 1 | 1% | 4% | 4 |
| Donor # 2 | 1 % | 7% | 7 |
| Donor # 3 | 6% | 15% | 2.5 |
| Mean ± SD | 2.7 ± 2.9 | 8.7±5.7 | 4.5 ± 2.3 |

**Table 2: Enrichment of CD34⁺38⁻, early stem cell population in cultrue in absence and presence of cytostatic agent. Results obtained after expansion of CD133 positive cells for one week in the absence or presence of cytostatic agent (colchicine) at 2.5 ng/ml.**

| Experiment | CD34+38- cell population *without* cytostatic agent | CD34+38- cell population *with* cytostatic agent | Fold enrichment |
|---|---|---|---|
| Donor # 3 | 9% | 15% | 1.7 |
| Donor # 4 | 0.5% | 5% | 10 |

**Table 3: Enhanced stem cells expansion by cytostatic agent. Fresh, enriched CD133⁺ stem cells from various donors were plated at densities indicated at To. Counts of total cells and CD133⁺ cells are shown after 2 weeks of ex-vivo expansion in the absence or presence of cytostatic agent (colchicine at 2.5 ng/ml). Fold expansion is shown in parentheses.**

| Exp. | T₀ | T_{(2 weeks)} | | | |
|---|---|---|---|---|---|
| | CD133+ cells | Without colchicine | | With 2.5 ng.ml colchicine | |
| | | Total cells | CD133+ cells | Total cells | CD133+ cells |
| 1 | 0.4x10³ | 270x10³ | 2.7x10³ | 135x10³ | 5.4x10³ |
| | | | (X 6.5) | | (X 13.5) |
| 2 | 0.2x10³ | 135x10³ | 1.3x10³ | 68x10³ | 4.7x10³ |
| | | | (X 6.5) | | (X 23.5) |
| 3 | 0.4x10³ | 81x10³ | 4.9x10³ | 27x10³ | 4.1x10³ |
| | | | (X 12.3) | | (X 10.2) |

Based on the abovementioned results, the invention provides a process for the selection, enrichment and expansion of stem/progenitor cells subsets that are commonly a constituent of umbilical cord blood, peripheral blood, and bone marrow. The process could be also suitable for selective enrichment and expansion of other types of stem/progenitor cells that predominantly express Pgp (MDR1).

While the invention has been described with respect to a limited number of embodiments, it will be appreciated that many variations, modifications and other applications of the invention may be made.

### References

Abbott BL. ABCG2 (BCRP) expression in nominal and malignant hematopoietic cells. Hematol Oncol. 2003, 21:115-30. Review.
Ambudkar SV, Kimchi-Sarfaty C, Sauna ZE, Gottesman MM. P-glycoprotein: from genomics to mechanism. Oncogene. 2003, 22:7468-85. Review.
Benito AI, Diaz MA, Gonzalez-Vicent M, Sevilla J, Madero L. Hematopoietic stem cell transplantation using umbilical cord blood progenitors: review of current clinical results. Bone Marrow Transplant. 2004, 33:675-90.
Bertolini F, Battaglia M, Lanza A, Gibelli N, Palermo B, Pavesi L, Caprotti M, Robustelli della Cuna G. Multilineage long-term engraftment potential of drug-resistant hematopoietic progenitors. Blood. 1997, 90:3027-36.
Bertoncello I, Williams B. Hematopoietic stem cell characterization by hoechst 33342 and rhodamine 123 staining. Methods Mol Biol. 2004;263:181-200.
Chopp M and Y Li. Treatment of neural injury with marrow stromal cells. Lancet Neurol. 2002 1:92-100.
Cohen Y, Nagler A. Cord blood biology and transplantation. Isr Med Assoc J. 2004, 6:39-46. Review.
Devine SM, Lazarus HM, Emerson SG. Clinical application of hematopoietic progenitor cell expansion: current status and future prospects. Bone Marrow Transplant. 2003, 31:241-52. Review.
Dimitriou H, Vorgia P, Stiakaki E, Mavroudis D, Markaki EA, Koumantakis E, Kalmanti M. In vitro proliferative and differentiating characteristics of CD133(+) and CD34(+) cord blood cells in the presence ofthrombopoietin (TPO) or erythropoietin (EPO). Potential implications for hematopoietic cell transplantation. Leuk Res. 2003, 27:1143-51.
Handgretinger R, Gordon PR, Leimig T, Chen X, Buhring HJ, Niethammer D, Kuci S. Biology and plasticity of CD133+ hematopoietic stem cells. Ann N Y Acad Sci. 2003, 996:141-51.
Fiaccavento R, Carotenuto F, Minieri M, Fantini C, Forte G, Carbone A, Carosella L, Bei R, Masuelli L, Palumbo C, Modesti A, Prat M, Di Nardo P. Stem cell activation sustains hereditary hypertrophy in hamster cardiomyopathy. J Pathol. 2005, 205:397-407.
Findling-Kagan S, Sivan H, Ostrovsky O, Nagler A, Galski H. Establishment and characterization of new cellular lymphoma model expressing transgenic human MDR1. Leuk Res. 2005, 29:407-14.
Forraz N, Pettengell R, McGuckin CP. Characterization of a lineage-negative stemprogenitor cell population optimized for ex vivo expansion and enriched for LTC-IC. Stem Cells. 2004, 22:100-8.
Galski H, Nagler A. Novel inhibitors of multidrug resistance (MDR): Evaluation studies for future clinical applications. Ann Proc Eur Hematol 2003, 7:129
Gammaitoni L, Bruno S, Sanavio F, Gunetti M, Kollet O, Cavalloni G, Falda M, Fagioli F, Lapidot T, Aglietta M, Piacibello W. Ex vivo expansion of human adult stem cells capable of primary and secondary hemopoietic reconstitution. Exp Hematol. 2003, 31:261-70.
Iris B, Zilberman Y, Zeira E, Galun E, Honigman A, Turgeman G, Clemens T, Gazit Z, Gazit D. Molecular imaging of the skeleton: quantitative real-time bioluminescence monitoring gene expression in bone repair and development. J Bone Miner Res. 2003, 18:570-8.
Kasai M, Kiyama Y, Kawamura A. Application of peripheral blood stem cells (PBSC) mobilized by recombinant human granulocyte colony stimulating factor for allogeneic PBSC transplantation and the comparison of allogeneic PBSC transplantation and bone marrow transplantation. Transfus Apheresis Sci. 2002, 26:121-7. Review.
Koh LP, Chao NJ. Umbilical cord blood transplantation in adults using myeloablative and nonmyeloablative preparative regimens. Biol Blood Marrow Transplant. 2004, 10:1-22.
Koehl U, Zimmermann S, Esser R, Sorensen J, Gruttner HP, Duchscherer M, Seifried E, Klingebiel T, Schwabe D. Autologous transplantation of CD133 selected hematopoietic progenitor cells in a pediatric patient with relapsed leukemia. Bone Marrow Transplant. 2002,29:927-30.
Newman MB, Davis CD, Kuzmin-Nichols N, Sanberg PR. Human umbilical cord blood (HUCB) cells for central nervous system repair. Neurotox Res. 2003, 5:355-68. Review.
Peled T, Landau E, Prus E, Treves AJ, Fibach E. Cellular copper content modulates differentiation and self-renewal in cultures of coid-blood derived CD34+ cells. Brit. J. Haematol. 2002, 116:655-661
Perry TE, Roth SJ. Cardiovascular tissue engineering: constructing living tissue cardiac valves and blood vessels using bone marrow, umbilical cord blood, and peripheral blood cells. J Cardiovasc Nurs. 2003, 18:30-7. Review.
Petzer AL, Eaves CJ, Barnett MJ, Eaves AC. Selective expansion of primitive normal hematopoietic cells in cytokine-supplemented cultures of purified cells from patients with chronic myeloid leukemia. Blood. 1997, 90:64-9
Ros JE, Roskams TA, Geuken M, Havinga R, Splinter PL, Petersen BE, LaRusso NF, van der Kolk DM, Kuipers F, Faber KN, Muller M, Jansen PL. ATP binding cassette transporter gene expression in rat liver progenitor cells. 2003, Gut. 52:1060-7.
Sauna ZE, Smith MM, Muller M, Kerr KM, Ambudkar SV. The mechanism of action of multidrug-resistance-linked P-glycoprotein. J Bioenerg Biomembr. 2001, 33:481-91. Review.
Thomas J, Liu F, Link DC. Mechanisms of mobilization of hematopoietic progenitors with granulocyte colony-stimulating factor.Curr Opin Hematol. 2002, 9:183-9. Review.
Uchida N, Dykstra B, Lyons K, Leung F, Kristiansen M, Eaves C. ABC transporter activities of murine hematopoietic stem cells vary according to their developmental and activation status. 2004, Blood 103:4487-95.
Wagner W, Ansorge A, Wirkner U, Eckstein V, Schwager C, Blake J, Miesala K, Selig J, Saffrich R, Ansorge W, Ho AD. Molecular evidence for stem cell function of the slow-dividing fraction among human hematopoietic progenitor cells by genome-wide analysis. Blood. 2004,104:675-86.
Zhou S, Schuetz JD, Bunting KD, Colapietro AM, Sampath J, Morris JJ, Lagutina I, Grosveld GC, Osawa M, Nakauchi H, Sorrentino BP. The ABC transporter Bcrp1/ABCG2 is expressed in a wide variety of stem cells and is a molecular determinant of the side-population phenotype. Nat Med. 2001, 7:1028-34.

## Claims

1. An in vitro method for producing a population enriched for stem/progenitor cells, the population including stem/progenitor cells that inherently overexpress at least one transporter gene selected from the group consisting of an ABCB1 (MDR1) gene and/or an ABCG2 gene and/or MRP1 gene and/or MRP2 gene and/or MRP3 gene relative to the more differentiated progenitor and mature cells of the population, said method comprising:
treating said population with at least one first agent to which stem/progenitor cells are less sensitive than are other cells in said population, said first agent selected from the group consisting of cytostatic agents and cytotoxic agents, said first agent selectively depleting from the population said more differentiated progenitor and mature cells, thereby producing a population enriched for stem/progenitor cells, wherein said population is not obtained from a human embryonic source.

2. The method of claim 1, wherein said population is obtained from a non-human embryonic source.

3. The method of claim 1, wherein said population is obtained from a fetal source.

4. The method of claim 1, wherein said population is obtained from an adult source.

5. The method of claim 1, wherein said population is obtained from a source selected from the group consisting of bone marrow, blood, fat, muscle, skin, tissues of an internal organ and neural tissues.

6. The method of claim 5, wherein said blood is peripheral blood or umbilical cord blood.

7. The method of claim 5 wherein said internal organ is selected from the group consisting of heart, intestine, kidney, liver, lung and pancreas.

8. The method of claim 1, further comprising the step of treating said population with at least one second agent selected from the group consisting of cytokines and growth factors.

9. The method of claim 1 for performing expansion of a stem/progenitor cell population having a phenotype, selected from the group consisting of at least one of CD133⁺ and CD34⁺38⁻ phenotype in a population of cells.

10. A pharmaceutical composition comprising a therapeutically effective amount of the cell population of claim 1.

11. A composition of claim 10 for use in:
(i) bone marrow transplantation;
(ii) adoptive immunotherapy;
(iii) reconstitution of hematopoiesis after myeloablative therapy;
(iv) autologous and allogeneic transplantations;
(v) treatment of cardiac insufficiency;
(vi) cell-based transplantation therapies for neurodegenerative disorders; or
(vii) cell transplantation and/or tissue engineering.

## Patentansprüche

1. Ein In-vitro-Verfahren zur Erzeugung einer Population, die im Hinblick auf Stamm-/Vorläuferzellen angereichert ist, wobei die Population Stamm-/Vorläuferzellen einschließt, welche mindestens ein Transporter-Gen inhärent überexprimieren, das gewählt ist aus der Gruppe bestehend aus einem ABCB1-(MDR1-)Gen und/oder einem ABCG2-Gen und/oder MRP1-Gen und/oder MRP2-Gen und/oder MRP3-Gen mit Bezug auf die differenzierteren Vorläufer- und reifen Zellen der Population, wobei das Verfahren Folgendes umfasst:
Behandlung der Population mit mindestens einem ersten Mittel, auf das Stamm-/Vorläuferzellen weniger empfindlich reagieren als andere Zellen in der Population, wobei das erste Mittel gewählt ist aus der Gruppe bestehend aus Zytostatika und Zytotoxika, wobei das erste Mittel die differenzierteren Vorläufer- und reifen Zellen selektiv aus der Population beseitigt, wodurch eine Population erzeugt wird, die im Hinblick auf Stamm-/Vorläuferzellen angereichert ist, worin die Population nicht aus einer human-embryonalen Quelle gewonnen wird.

2. Das Verfahren gemäß Anspruch 1, worin die Population aus einer nicht-human-embryonalen Quelle gewonnen wird.

3. Das Verfahren gemäß Anspruch 1, worin die Population aus einer fetalen Quelle gewonnen wird.

4. Das Verfahren gemäß Anspruch 1, worin die Population aus einer adulten Quelle gewonnen wird.

5. Das Verfahren gemäß Anspruch 1, worin die Population aus einer Quelle gewonnen wird, die gewählt ist aus der Gruppe bestehend aus Knochenmark, Blut, Fett, Muskel, Haut, Geweben eines inneren Organs und Nervengeweben.

6. Das Verfahren gemäß Anspruch 5, worin das Blut peripheres Blut oder Nabelschnurblut ist.

7. Das Verfahren gemäß Anspruch 5, worin das innere Organ gewählt ist aus der Gruppe bestehend aus Herz, Darm, Niere, Leber, Lunge und Bauchspeicheldrüse.

8. Das Verfahren gemäß Anspruch 1, das weiter den Schritt der Behandlung der Population mit mindestens einem zweiten Mittel umfasst, das gewählt ist aus der Gruppe bestehend aus Zytokinen und Wachstumsfaktoren.

9. Das Verfahren gemäß Anspruch 1 zur Durchführung der Expansion einer Stamm-/Vorläuferzellpopulation mit einem Phänotyp, der gewählt ist aus der Gruppe bestehend aus mindestens einem des CD133⁺- und des CD34⁺38⁻-Phänotyps in einer Population von Zellen.

10. Eine pharmazeutische Zusammensetzung, die eine therapeutisch wirksame Menge der Zellpopulation gemäß Anspruch 1 umfasst.

11. Eine Zusammensetzung gemäß Anspruch 10 zur Verwendung in:
(i) Knochenmarktransplantation,
(ii) adoptiver Immuntherapie,
(iii) Rekonstitution von Hämatopoese nach myeloablativer Therapie,
(iv) autologen und allogenen Transplantationen,
(v) Behandlung von Herzinsuffizienz,
(vi) zellbasierten Transplantationstherapien für neurodegenerative Störungen oder
(vii) Zelltransplantation und/oder Gewebezüchtung.

## Revendications

1. Procédé in vitro de production d'une population enrichie de cellules souches/cellules progénitrices, la population comprenant des cellules souches/cellules progénitrices surexprimant de manière inhérente au moins un gène transporteur, sélectionné dans le groupe composé d'un gène ABCB1 (MDR1) et/ou d'un gène ABCG2 et/ou d'un gène MRP1 et/ou d'un gène MRP2 et/ou d'un gène MRP3 relativement aux cellules progénitrices plus différenciées et matures de la population, ledit procédé comprenant :
le traitement de ladite population avec au moins un premier agent envers lequel des cellules souches/cellules progénitrices sont moins sensibles que le sont d'autres cellules dans ladite population, ledit premier agent étant sélectionné dans le groupe composé d'agents cytostatiques et d'agent cytotoxiques, ledit premier agent soustrayant sélectivement de la population lesdites cellules progénitrices plus différenciées et matures de la population, de manière à produire une population enrichie en cellules souches/cellules progénitrices, dans lequel ladite population n'est pas obtenue à partir d'une source embryonnaire humaine.

2. Procédé selon la revendication 1, dans lequel ladite population est obtenue à partir d'une source embryonnaire non humaine.

3. Procédé selon la revendication 1, dans lequel ladite population est obtenue à partir d'une source foetale.

4. Procédé selon la revendication 1, dans lequel ladite population est obtenue à partir d'une source adulte.

5. Procédé selon la revendication 1, dans lequel ladite population est obtenue à partir d'une source sélectionnée dans le groupe composé de moelle osseuse, sang, graisse, muscle, peau, tissus d'un organe interne et tissus neuronaux.

6. Procédé selon la revendication 5, dans lequel ledit sang est du sang périphérique ou du sang de cordon ombilical.

7. Procédé selon la revendication 5, dans lequel ledit organe interne est sélectionné dans le groupe composé du coeur, intestin, rein, foie, poumon et pancréas.

8. Procédé selon la revendication 1, comprenant en outre l'étape de traitement de ladite population avec au moins un deuxième agent, sélectionné dans le groupe compos de cytokines et facteurs de croissance.

9. Procédé selon la revendication 1, pour effectuer une expansion d'une population de cellules souches/cellules progénitrices présentant un phénotype sélectionné dans le groupe composé d'au moins un phénotype CD133⁺ et CD34⁺38⁻ dans une population de cellules.

10. composition pharmaceutique, comprenant une quantité thérapeutiquement efficace de la population de cellules de la revendication 1.

11. Composition selon la revendication 10, pour utilisation dans :
(i) une transplantation de moelle osseuse ;
(ii) une immunothérapie adoptive ;
(iii) une reconstitution hématopoïétique après une thérapie myéloablative ;
(iv) des transplantations autologues et allogènes ;
(v) un traitement contre l'insuffisance cardiaque ;
(vi) des thérapies à transplantation à base de cellules pour les troubles neurodégénératifs ; ou
(vii) transplantations de cellules et/ou ingénierie tissulaire.
